# EUROPEAN PATENT APPLICATION

(11) **EP 1 983 338 A2**
(43) Date of publication of application: **22.10.2008**
(21) Application number: 08156676.2
(22) Date of filing: 21.05.2008
(51) Int. Cl.: G01N 25/28

(54) **Method for monitoring emissions of NOx from an emission source**

(71) Applicant: StatoilHydro ASA, 4035 Stavanger (NO)
(72) Inventor: Valdersnes, Trond, N-5254, Sandsli (NO); Torsvik, Jone, N-5251, Søreidgrend (NO)
(74) Representative: Lajer, Dorte

(57) **Abstract**

The present invention relates to a method for monitoring emissions of NOx from an emission source. Said method is a more cost efficient solution than CEMS/PEMS, and at the same time provide a far better estimate of NOx emissions than today's "factor method".

## Description

### FIELD OF THE INVENTION

The present invention relates to a method for monitoring emissions of nitrogen oxides (NOx) from an emission source. The invention also relates to a computer system adapted to implement said method, a parametric emissions monitoring system adapted to perform said method and a mechanical power generating system comprising an emission source and said parametric emission monitoring system.

### BACKGROUND OF THE INVENTION

Public awareness has increased with respect to the environment, and primary pollutants such as nitrogen oxides and sulfur dioxide are currently regulated in most industries. A great deal of attention in recent years has been spent on addressing the monitoring requirements of these regulations, in order to minimize the discharge of noxious gases into the atmosphere by industrial facilities.

One technique for ensuring correct monitoring of NOx has been to implement continuous emissions monitoring systems (CEMS). A CEM system typically includes a gas analyzer installed either directly in the exhaust stack, or connected via an extractive system which extracts a gas sample from the exhaust stack and conveys it to an analyzer at grade level. Continuous emissions monitoring systems are quite expensive, particularly due to the installation cost and demanding maintenance and calibration requirements of the gas analyzers.

In order to target the challenges associated with CEMS, solutions have been developed that does away with the gas analyser and instead uses a computer based model for predicting the NOx emissions. The model uses as input a number of monitored parameters from the energy or fuel conversion process, such as temperatures and pressures. These systems are referred to as predictive or parametric emissions measurement systems (PEMS).

There have been PEMS built in the past to predict various combustion and emission parameters from continuous industrial processes and to calculate process or combustion efficiency for compliance reporting and process optimization purposes. Typically, the PEMS is "trained" by monitoring multiple inputs such as pressures, temperatures, flow rates, etc., and one or more output parameters such as NOₓ, CO, O₂, etc. After training, in normal operation, the PEMS monitors only the multiple inputs and calculates estimated output parameter values that closely match the actual pollutant levels. Methodologies used in the past include nonlinear statistical, neural network, eigenvalue, stochastic, and other methods of processing the input parameters from available field devices and to predict process emission rates and combustion or process efficiency. For the most part, these PEMS are complicated, relatively costly, and difficult to implement. These systems also typically require retraining with the support of specialized staff from the system provider to adjust the proprietary model to the real-world conditions encountered in the field.

Since the majority of e.g. (offshore) mechanical power installations do not have equipment or methods in place for monitoring NOx emissions, these will have to be determined by simpler methods. One such method is the "factor method", whereby the amount of fuel converted to mechanical energy is multiplied by a general or source specific factor expressing NOx [kg]/fuel unit. The main weakness of this method is that it does not correct for variations in specific NOx emissions as a function of variations in the energy or fuel conversion process.

Provided a regulatory acceptance of methods for NOx quantification that does not present the same documentable accuracy as that of of CEMS and PEMS, it is of interest to arrive at an alternative solution which is simpler, faster and more cost efficient with respect to installation and maintenance, yet reliable and sufficient to compensate for the changes in the energy or fuel conversion process.

### SUMMARY OF THE INVENTION

The object of the present invention is to contribute to the set up of a simple, reliable and more cost efficient solution than CEMS/PEMS, and at the same time provide a far better estimate of NOx emissions than today's "factor method".

Thus, a first aspect of the present invention relates to a method for monitoring emissions of NOx from an emission source, wherein said method comprises the following steps:
- measure the exhaust gas temperature;
- look up the corresponding NOx emissions data from a dataset which is representative for said emission source, either as a mass figure or as a concentration that can be converted to mass by the use of a conversion routine.

The dataset representative for said emission source may be obtained by measuring the exhaust gas composition at different exhaust gas temperatures. Fuel and/or air flow can be measured together with fuel gas composition and a conversion routine be employed in order to include NOx emissions on a mass basis in the data set. Other process parameters can be included in the data set order to provide a multi-variable model.

A second aspect of the present invention relates to a parametric emission monitoring system adapted to perform a method in accordance with the first aspect of the present invention.

A third aspect of the present invention relates to a mechanical power generating system comprising:
- an emission source; and
- a parametric emission monitoring system according to the second aspect of the present invention.

### DESCRIPTION OF FIGURES

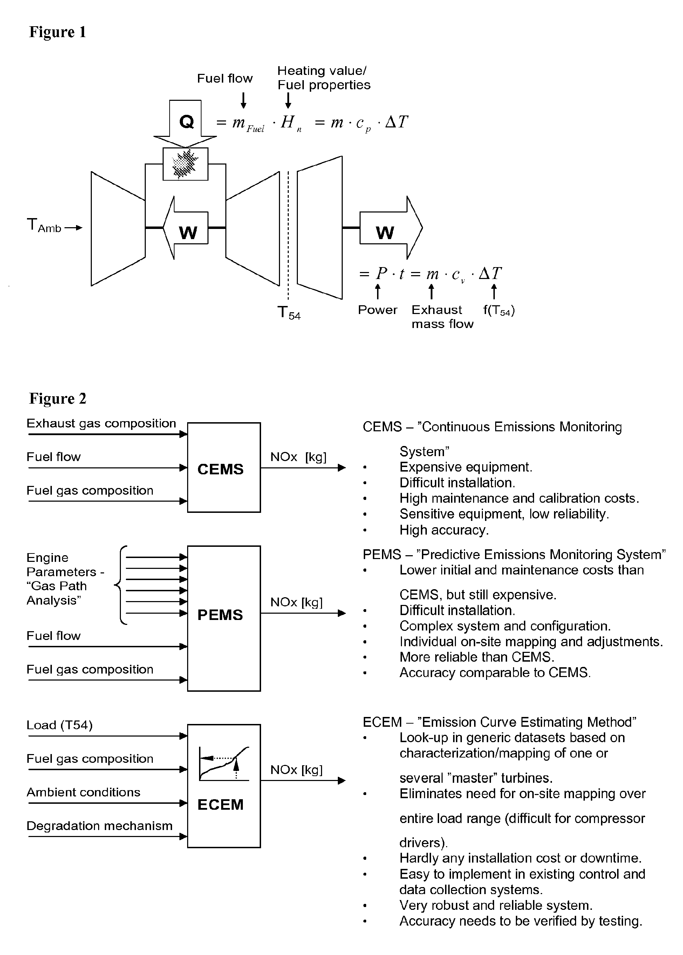

**Figure 1** - Ilustrates basic relationships between various parameters in a combustion chamber.
**Figure 2** - Methods of NOx quantification

### DETAILED DESCRIPTION OF THE INVENTION

A gas turbine is a thermodynamic machine where the chemical energy in fuel is released through combustion in compressed air. The enthalpy in the hot gas resulting from the combustion is converted into mechanical energy through thermal expansion in several stages through a turbine. A part of the mechanical work involves driving the compressor which supplies the combustion chamber with compressed air. The rest is available as "shaft power" for the driving of generators or large process gas compressors.

NOx is formed in the flame zone itself where the temperature is so high that molecular nitrogen is oxidised, i.e. it reacts with oxygen to form NOx. These are complex processes difficult to predict and/or simulate.

For a given machine configuration working as it is supposed to, the present inventors have observed a very good correlation between the power/load and the flame temperature, and consequently also the NOx formation. However in contrast to the power/load, the exhaust gas temperature is universally accessible for all turbines as an important monitored parameter, and thus ideal as input for a look-up model.

Accordingly, a first aspect of the present invention relates to a method for monitoring emissions of NOx from an emission source, wherein said method comprises the following steps:
- measure the exhaust gas temperature;
- look up the corresponding NOx emissions data from a dataset which is representative for said emission source, either as a mass figure or as a concentration that can be converted to mass by the use of a conversion routine.

Other parameters assumed to impact significantly on NOx emissions specific to the amount of fuel consumed :
- fuel composition and heating value
- ambient air temperature and humidity (since the machine is operating with an open cycle)
- any parameter sensitive to differences in - and changes to - the turbine characteristics itself, e.g. through degradation between overhauls.

Accordingly, the method according to the first aspect of the present invention may also include measurements of other parameters such as: fuel composition, ambient air temperature and humidity. In case the correlation between NOx emissions and any of the used parameters is not readily obtained by testing; simulated, calculated or empirical data may be used.

The approach of the present invention is somewhat different than that of PEMS, where the basic is to replicate the accuracy level CEMS by replacing the emissions measurement with a sophisticated model calibrated for the individual turbine in question. The approach of the present invention is rather to exploit the improvement potential of the "factor method" that lies in a continuous correction for operating point, with a possibility of increasing precision by including further a limited number of important parameters impacting on the formation of NOx.

Statoilhydro's tentative implementation of the method according to the present invention suggests that NOx figures, based on source specific factors, are over-reported by 30%. If this proves representative for more of the installations, it means that the method according to the present invention represents a quantum jump compared to the factor method as far as accuracy is concerned.

## Claims

1. A method for monitoring emissions of NOx from an emission source, wherein said method comprises the following steps:
- measure the exhaust gas temperature;
- look up the corresponding NOx emissions data from a dataset which is representative for said emission source, either as a mass figure or as a concentration that can be converted to mass by the use of a conversion routine.

2. The method of claim 1, wherein said dataset has been obtained by measuring the exhaust gas composition, preferably also the fuel and/or air flow, at different exhaust gas temperatures.

3. A computer system adapted to implement a method in accordance with claim 1 or 2.

4. A parametric emission monitoring system adapted to perform a method in accordance with claim 1 or 2.

5. A mechanical power generating system comprising:
- an emission source; and
- a parametric emission monitoring system according to claim 4.
